# FASCICULE DE BREVET EUROPEEN

(11) **EP 0 719 752 B1**
(45) Date de publication et mention de la délivrance du brevet: **12.03.2008**
(21) Numéro de dépôt: 95402948.4
(22) Date de dépôt: 27.12.1995
(51) Int. Cl.: C07C 43/13, C07C 69/33, C07C 41/09

(54) **Procédé pour l'obtention de polyglycérols et d'esters de polyglycérols**
Verfahren zur Herstellung von Polyglycerolen und Polyglycerolestern
Process for producing polyglycerols and polyglycerol esters

(30) Priorité: 29.12.1994 FR 9415834
(43) Date de publication de la demande: 03.07.1996
(73) Titulaire: Institut Français du Pétrole, 92502 Rueil-Malmaison (FR)
(72) Inventeur: Durand, Isabelle, F-92500 Rueil Malmaison (FR); Hillion, Gérard, F-95220 Herblay (FR); Stern, Robert, F-75017 Paris (FR)

(56) Documents cités:
- DE-A- 3 346 097
- FR-A- 2 677 643
- US-A- 5 147 644
- US-A- 5 247 114

## Description

La présente invention a pour objet un procédé pour l'obtention de polyglycérols ainsi que d'esters de polyglycérols.

Il est connu de fabriquer des polyglycérols, qui sont des polyéthers de glycérine, par chauffage de glycérol pur en présence de catalyseurs basiques ou acides. De même, on sait préparer des esters de polyglycérols par action d'acides gras, d'esters ou d'huiles sur des polyglycérols. Ces procédés sont connus depuis longtemps.

Les polyglycérols sont des liquides visqueux ayant la propriété de retenir l'eau : ils peuvent servir d'humectants, d'agents antistatiques, d'antimottants, de solvants, mais surtout de matière première pour la synthèse d'esters de polyglycérols.

Ces derniers peuvent être utilisés dans de nombreuses industries, où, selon leur degré de substitution, on les emploie comme émulsifiants, antimoussants, lubrifiants et même plastifiants.

Le seul handicap dont souffrent ces produits, est leur prix, qui dépend de la matière première. Le brevet français FR-B-2677643 décrit l'utilisation comme matière première soit de la glycérine brute encore basique issue d'une fabrication d'esters (par exemple méthyliques) de corps gras à partir d'huiles, soit de la même phase alcaline glycérineuse après neutralisation par exemple par l'acide acétique ou autre acide carboxylique faible.

S'il est vrai que cette glycérine brute est beaucoup moins chère que la glycérine distillée, il reste qu'elle n'est utilisable que sur le site de fabrication compte tenu que le méthanol qu'elle contient doit être recyclé dans le procédé. La concentration de l'ordre de 40 % en glycérine de ces solutions en rend le transport peu rentable.

Une amélioration substantielle du procédé consisterait à partir d'une glycérine technique contenant comme impuretés uniquement de l'eau et des sels d'acides minéraux comme le chlorure de sodium, le sulfate de sodium ou le phosphate de sodium. Ces glycérines sont en effet commerciales, elles ne contiennent pas de méthanol et titrent généralement de 65 à 85 % en glycérine pure. Les spécifications en matières organiques non glycérineuses des glycérines techniques ne dépassant pas 2,5 %.

L'invention a pour objet un nouveau procédé de fabrication de polyglycérols et d'esters de polyglycérols permettant d'obtenir ces produits de manière plus simple et plus économique que dans l'art antérieur.

Le procédé de l'invention fournit des polyglycérols qui, de manière surprenante, ont une coloration équivalente à celle de produits analogues fabriqués par des procédés connus à partir de glycérine distillée et, de plus, ces polyglycérols se laissent facilement épurer jusqu'à l'obtention de produits incolores, ceci par des procédés simples, comme par exemple le passage sur des résines échangeuses d'ions.

D'une manière générale, l'invention à pour objet un procédé pour l'obtention de polyglycérols et d'esters de polyglycérols comprenant principalement une étape de chauffage en présence d'au moins une base, d'une matière première glycérineuse contenant au moins un sel minéral tel qu'un sulfate, chlorure ou phosphate de métal alcalin, par exemple de sodium. Une telle matière première peut provenir d'une réaction de transestérification d'huiles ou de la fabrication de savons.

La transestérification des huiles avec des alcools à bas poids moléculaire qui est à l'origine de la phase glycérineuse peut se réaliser à partir d'huiles végétales ou animales, dont on peut citer comme exemples : les huiles de palmiste, de palme, de coprah, de babassu, de tournesol, de colza érucique, de canola, de lin, d'arachide, de coton, de carthame, de poisson, de soja ou de tall oil ; le suif, le saindoux, ou même des huiles de friture. La glycérine de savonnerie se fabrique généralement à partir de mélange de suif, d'huile de palme, d'huile de palmiste ou d'huile de coton.

Dans le cas de la transestérification, les huiles sont transestérifiées en présence d'alcools divers : méthanol, éthanol, butanol ou isopropanol, utilisés seuls ou en mélange.

Les phases glycérineuses obtenues dans un premier temps dans le procédé de transestérification basique, contiennent la majorité des ions alcalins provenant des catalyseurs : bases alcalines fortes (soude ou potasse), carbonates ou alcoolates de métaux alcalins. Les solutions glycérineuses sont d'abord neutralisées, de préférence avec de l'acide sulfurique, chlorhydrique ou phosphorique, et ensuite soumises à une évaporation de l'alcool, qui permet une séparation après décantation des acides gras et esters entraînés dans les solutions glycérineuses. Selon les cas, on les concentre en évitant que les sels, sulfates, chlorures ou phosphates, ne cristallisent. Ces opérations sont couramment pratiquées dans les usines fabriquant les esters méthyliques ou éthyliques.

Avant de procéder à la polycondensation, on peut traiter la glycérine chargée en sels minéraux de départ, pour en éliminer certaines substances organiques en suspension ou en émulsion. Ce traitement fait appel à du charbon actif, des terres actives ou à des agents de coalescence adaptés. Ce traitement peut avoir pour effet la décoloration de la glycérine.

Les manières de polycondenser la glycérine selon le procédé de l'invention sont diverses, mais les premières étapes sont généralement communes. On chauffe les phases glycérineuses chargées en sels qui contiennent de 50 à 90 % de glycérine en présence d'une base alcaline. Pour éviter la formation de mousses, il convient d'évaporer le maximum d'eau du mélange avant d'introduire à 130 - 140 °C la base qui servira de catalyseur. Jusqu'à 220 - 230 °C, on recueille simplement l'eau encore présente dans la glycérine et celle éventuellement introduite avec la base. Entre 230 et 270 °C, on recueille l'eau de condensation. Le sel présent n'interfère pas dans la mesure où il y a suffisamment d'ions basiques présents, par exemple de 0,3 à 2 % comptés en % poids de soude par rapport à la glycérine. Les bases sont principalement : la soude, la potasse, les alcoolates, les carbonates. On arrête la réaction lorsque le degré de polycondensation désiré est atteint. Celui-ci est déterminé par la mesure du volume d'eau recueilli. Avant d'effectuer le chauffage de la solution glycérineuse, il est important de s'assurer que cette solution ne présente pas une valeur de pH acide, ce qui pourrait provoquer une décomposition de la glycérine pour former de l'acroléine.

Si l'on s'arrête à la fabrication de polyglycérols (voir plus loin schéma 1), on refroidit à 150 °C ou en-dessous pour effectuer la neutralisation de la base à l'aide d'acide sulfurique, chlorhydrique ou phosphorique.

Dans certains cas, pour diminuer la teneur résiduelle en glycérine du mélange de polyglycérols obtenu (par exemple à moins de 3 % en poids), on peut avoir avantage à opérer le refroidissement du mélange réactionnel de façon rapide (par exemple en quelques minutes) jusqu'à une température d'environ 220°C tout en procédant à une élimination poussée de l'eau de condensation résiduelle, par exemple sous un vide dynamique ou par entraînement au moyen d'un gaz inerte. On refroidit ensuite à 150°C ou en dessous. On a en effet observé qu'une teneur d'environ 1 % en poids d'eau dans le milieu réactionnel pouvait conduire à la présence d'environ 10 % en poids de glycérine dans le mélange formé, dans les conditions de la condensation.

On peut noter, à cet égard, que ces dispositions opératoires (élimination poussée de l'eau de condensation et refroidissement rapide du mélange réactionnel) peuvent être mises en oeuvre avantageusement également dans le cas où l'on prépare les polyglycérols à partir de glycérine pure et non de glycérine technique.

Lorsque l'on part d'une glycérine sulfatée, la neutralisation à l'acide sulfurique est préférée. Le sel de sodium ainsi obtenu est alors cristallisé par addition à environ 120 °C d'un volume d'alcool équivalent à la solution de polyglycérols. La filtration du sulfate ou du phosphate alcalin se fait sans problème. Le chlorure de sodium, par contre, est un peu plus soluble dans l'alcool. Comme alcool, on peut utiliser notamment le méthanol, l'éthanol, l'isopropanol ou le n-butanol. Les alcools sont recyclés et servent continuellement à diluer le polyglycérol afin de précipiter les sels; dans ce cas, le degré de pureté requis pour ces alcools est faible. Il reste que l'eau introduite par l'acide minéral utilisé peut s'accumuler lors des recyclages et rendre la précipitation des sels alcalins moins efficace. On a intérêt, lorsque l'on évapore l'alcool, à opérer avec une colonne à reflux, ou bien à utiliser un acide minéral peu chargé en eau. Après filtration du sulfate, chlorure ou phosphate et évaporation de l'alcool, on peut continuer l'épuration par un passage sur une colonne de résines cationiques, puis anioniques et ceci après dilution à l'eau du mélange de polyglycérols.

Afin d'éviter des pertes en polyglycérols, on peut laver les résines après utilisation par de l'eau désionisée avant leur régénération selon des protocoles connus. La phase aqueuse récupérée est alors évaporée et les polyglycérols ainsi obtenus sont réintroduits dans le procédé au niveau de l'étape de filtration du sel.

Plusieurs variantes existent pour la synthèse des esters de polyglycérols.

La première variante consiste à utiliser un polyglycérol pur obtenu selon le procédé décrit plus haut. On peut également éliminer par distillation (par exemple sous pression réduite) la glycérine libre et certains composés cycliques avant de réaliser la synthèse d'esters. Cette distillation se fait de préférence à l'aide d'un évaporateur à film tombant et râclant sous un vide poussé. Le polyglycérol qui ne contient plus, ou que peu, de glycérine (< 1 % poids), est ensuite soumis à l'estérification ou à la transestérification avec des acides gras (acides monocarboxyliques aliphatiques linéaires ou ramifiés, particulièrement à longue chaîne), des esters de tels acides avec des monoalcools aliphatiques, ou des huiles, selon des procédés connus, soit avec une base alcaline, soit avec un sel d'étain comme catalyseur (voir plus loin Schéma 2). Cette réaction se réalise en général à 170 - 250°C, de préférence 180 - 230°C, avec élimination de l'eau, du monoalcool ou du glycérol formé.

Un seconde variante utilise le polyglycérol purifié mais non passé sur résines, qui contient donc encore des sels alcalins.

Une troisième variante consiste à continuer la réaction de transestérification ou d'estérification à la suite de la polycondensation, en utilisant le même catalyseur (voir plus loin Schéma 3).

On refroidit donc à une température de 180 à 230°C, de préférence à environ 200°C, puis on introduit un acide gras ou un ester ; on laisse réagir, puis on refroidit et on neutralise entre 120 et 160°C, de préférence vers 150°C, très lentement au goutte à goutte avec un acide minéral fort comme par exemple l'acide sulfurique ou l'acide phosphorique. Pour filtrer le sulfate de sodium ou autres sels, on peut ajouter un solvant soit hydrocarboné soit alcoolique selon le degré de substitution des polyglycérols. Il est difficile d'obtenir lors de ces transestérifications ou estérifications une substitution totale. Lorsque le degré de substitution est faible, on observe généralement deux phases après la réaction : une phase polyglycérol qui n'a pas réagi et une phase esters de polyglycérol que l'on récupère par exemple par décantation avant de la purifier.

Si l'on arrive à substituer totalement le polyglycérol par des groupes esters, dans la mesure où l'on recherche un produit de ce type qui a des propriétés lubrifiantes, on peut laver à l'eau l'ester obtenu afin d'éliminer les sels alcalins lorsque l'indice d'hydroxyle de l'ester est assez faible pour que le produit ne forme pas d'émulsion. Ceci évite l'utilisation de solvants et permet ainsi une épuration bon marché (voir plus loin Schéma 3).

Comme il est difficile de faire réagir totalement tous les groupements hydroxyles libres et qu'il est également difficile de se débarrasser d'un excès d'acides gras on d'esters, on doit en général recourir à des solutions variées pour obtenir un ester sans groupements hydroxyles. Si l'on utilise des acides gras de faibles poids moléculaires, tel que par exemple l'acide 2 éthylhexanoïque, un excès peut être utilisé, qui sera distillé sans problème entre 180 et 230 °C, par exemple à environ 200°C, par exemple sous pression atmosphèrique. Sinon, il faut recourir à une distillation sous vide en présence de vapeur d'eau. On adoptera cette solution essentiellement sur un polyglycérol neutre. Pour terminer la réaction d'estérification, on recourt parfois à l'utilisation d'anhydride acétique.

Dans tous les cas, les polyglycérols fabriqués contiennent de la glycérine libre. Lorsque l'on effectue l'estérification immédiatement après la polycondensation, on fabrique comme sous-produits des glycérides partiels et même des triglycérides (huile). Dans la mesure où ces produits ne gènent pas dans l'utilisation projetée, on peut imaginer un procédé très simple comme indiqué en Schéma 4, qui consiste à utiliser une huile comme agent estérifiant. L'huile peut aussi être utilisée comme agent estérifiant dans le Schéma 3.

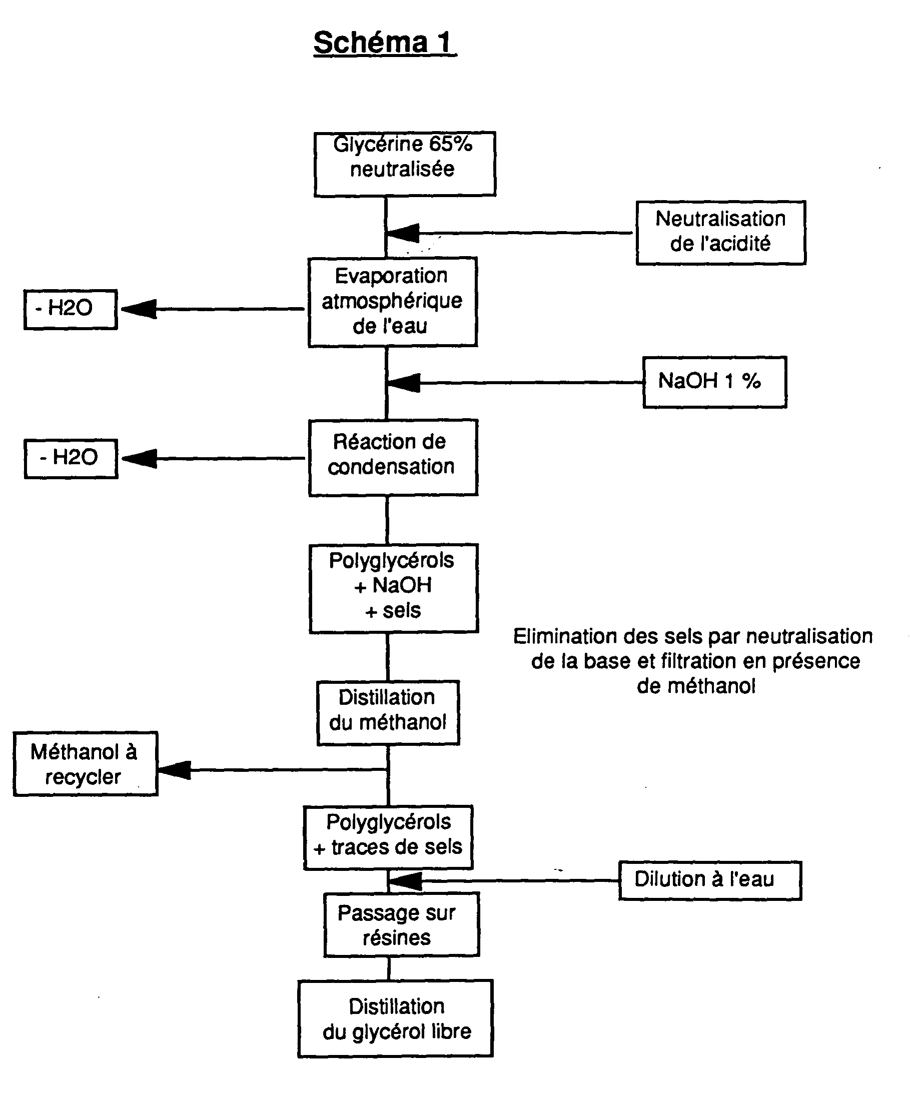

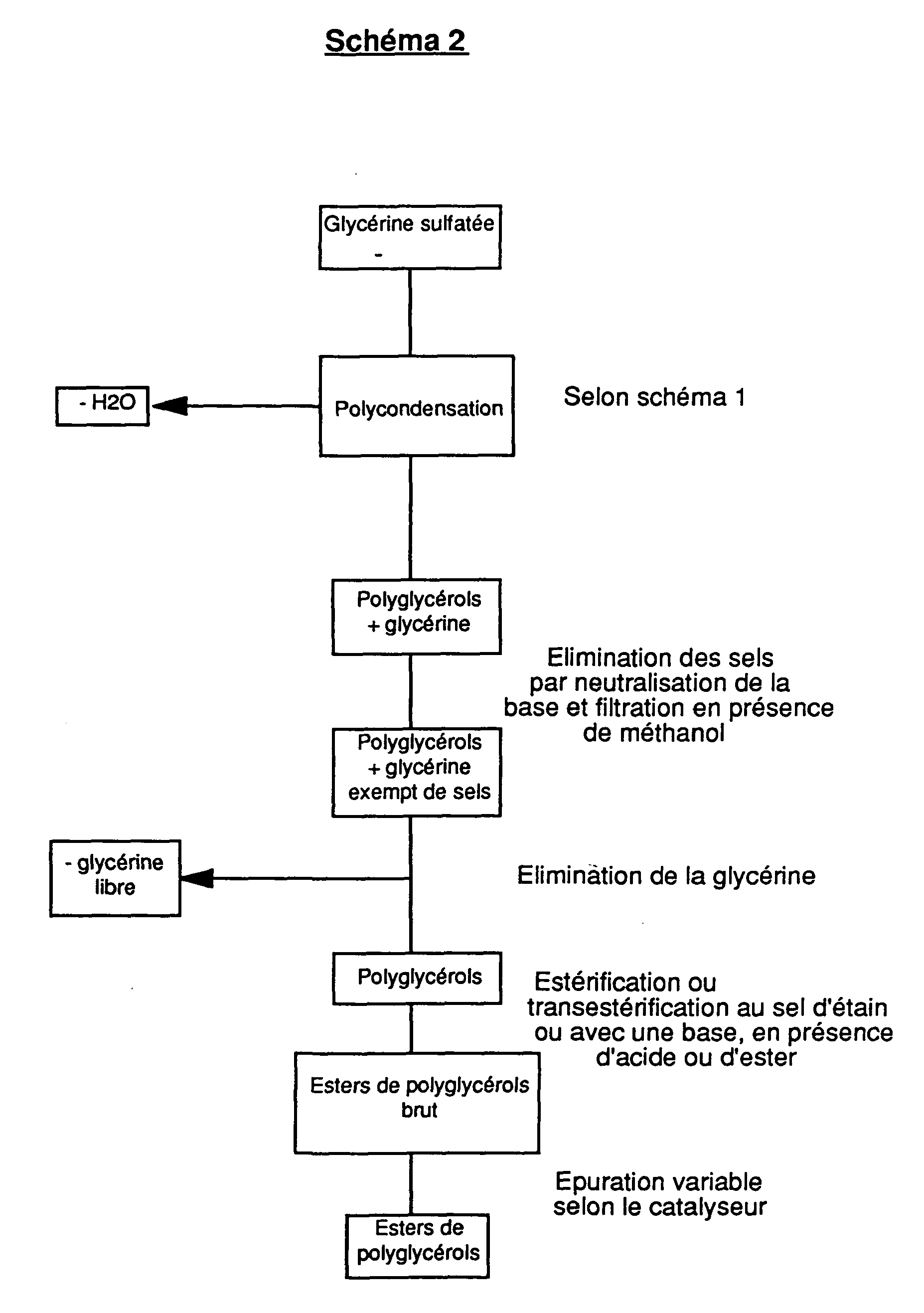

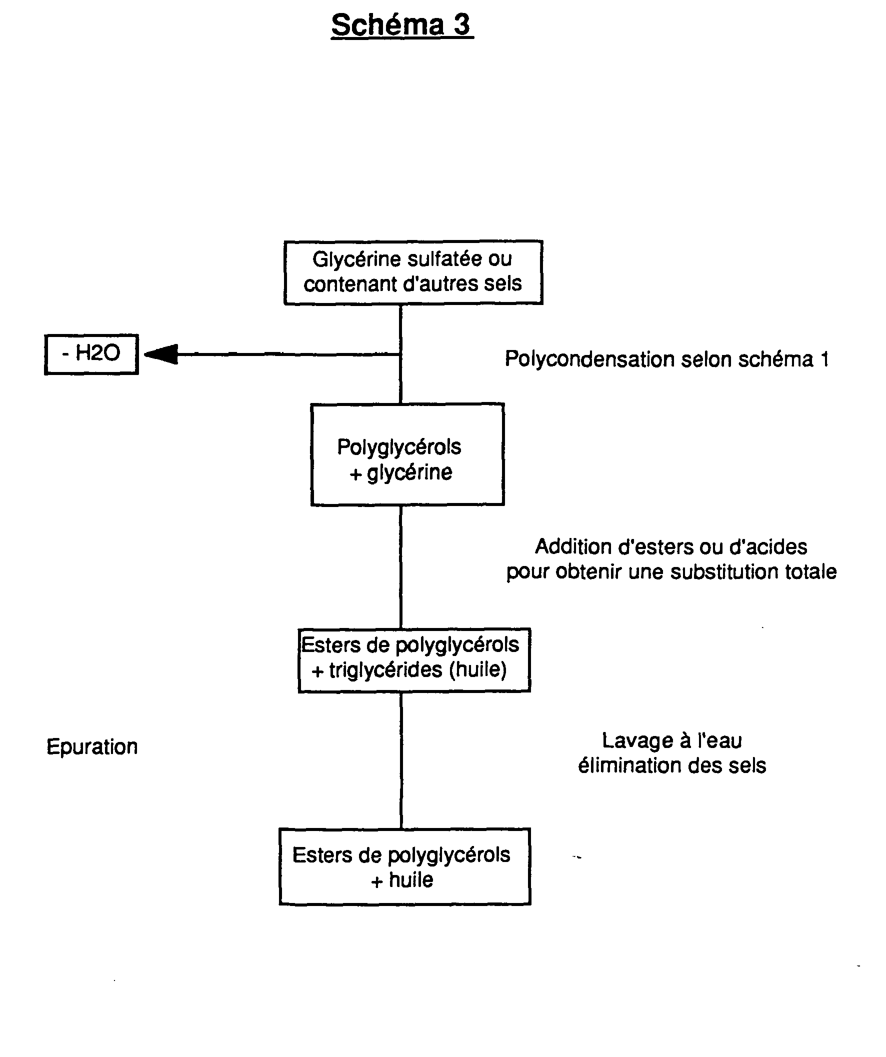

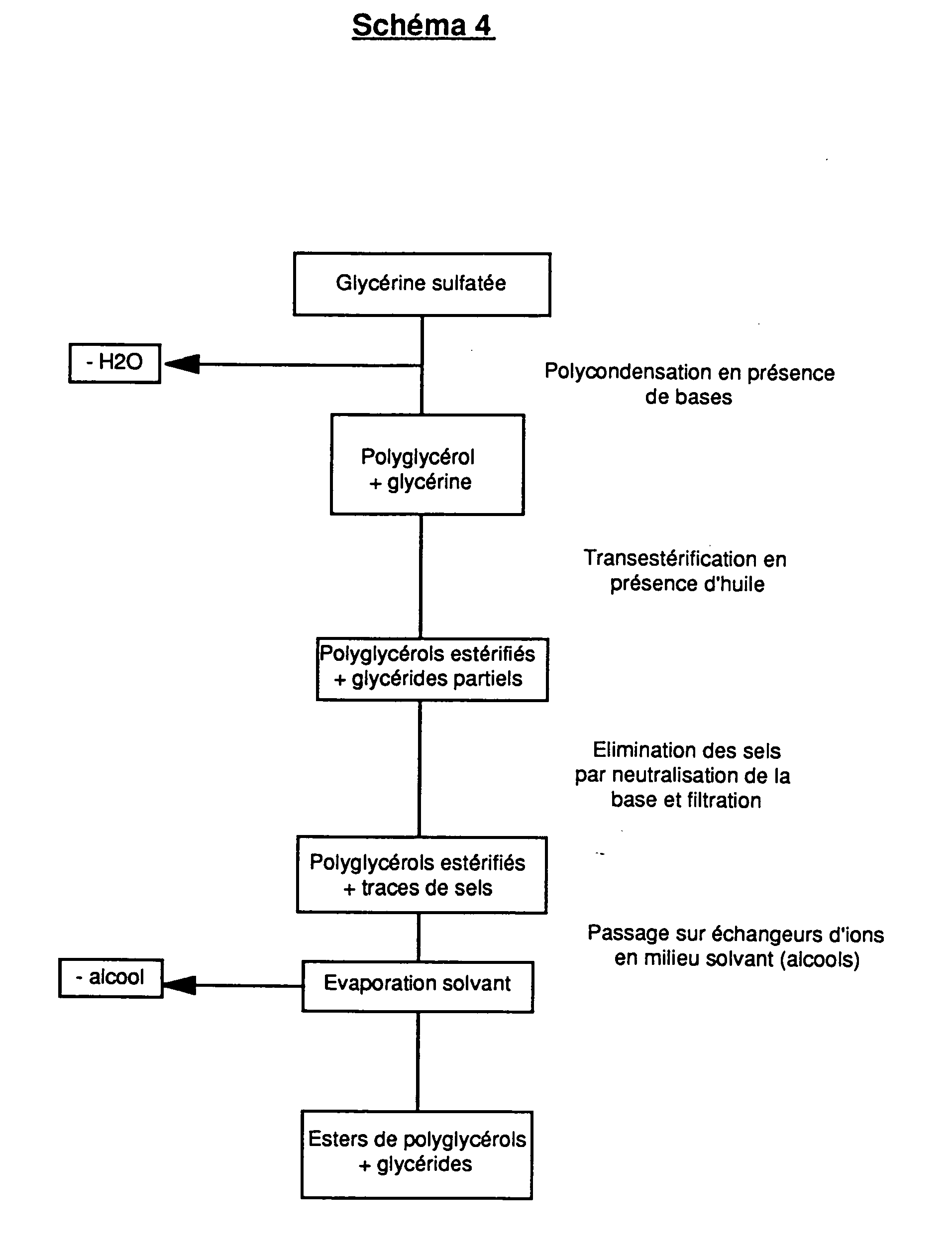

Les exemples suivants illustrent l'invention.

### EXEMPLE 1

### Fabrication de polyglycérols.

A 800 g de solution glycérineuse à 56 % en poids de glycérol et contenant environ 3,5 % en poids de sulfate de sodium, on ajoute 0,224 g de soude de manière à neutraliser la glycérine. Dans un ballon à reflux, on distille l'eau jusqu'à 130 - 140 °C en éliminant 311 g d'eau.

On ajoute alors 4,5 g de soude dans la glycérine. On monte progressivement la température. Il y a évaporation de l'eau résiduelle dès 170°C dans le ballon ou 104°C en haut du reflux. Après 15 minutes, la température de 263°C est atteinte. On obtient après 3 h à 265°C, 75 cm³ d'eau. La température est ramenée à 150 °C et l'on introduit goutte à goutte 11,1 g d'acide sulfurique à 50 % pour neutraliser la soude. On laisse refroidir à 120°C et l'on introduit lentement 238 cm³ de méthanol. Après 1/2 heure d'agitation à 50°C, on filtre le sulfate de sodium. Il reste dans le polyglycérol après évaporation du méthanol, 2850 ppm de sodium. Après passage sur une résine cationique puis une résine anionique du polyglycérol dilué dans un égal volume d'eau, on obtient 37 ppm de sodium résiduel dans le polyglycérol reconcentré.
L'analyse du polyglycérol est la suivante après silylation : (% en poids)

| | | |
|---|---|---|
| Glycérine | % | 23,80 |
| Diglycérols | % | 32,20 |
| Triglycérols | % | 19,65 |
| Tétraglycérols | % | 10,70 |
| Pentaglycérols | % | 4,97 |
| Hexaglycérols | % | 2,44 |
| Cycliques | % | 6,24 |

Ce produit est pratiquement incolore - Couleur Lovibond = 1

### EXEMPLE 2

### Fabrication de polyglycérols.

800 g de glycérine sulfatée contenant 65 % en poids de glycérol et titrant 0,67 mMole d'acide/100 g de solution, sont chauffés à 100°C dans un ballon sous un vide de 30 mm de Hg, afin d'éliminer l'eau du mélange. Il reste après évaporation 555,2 g de glycérine contenant 7,1 % en poids de sulfate de sodium et des traces d'eau. On ajoute 1 % en poids de soude + 0,217 g correspondant à la soude nécessaire à la neutralisation de l'acidité déterminée plus haut. A part 15 cm³ d'eau qui sont récupérés avant d'atteindre la température de 240°C, on recueille en 170 minutes et à 260°C, 67 g d'eau. On continue comme dans l'exemple 1 après avoir évaporé l'alcool et ajouté 50 % en poids d'eau par rapport au substrat obtenu avant le passage sur les résines. Le poids de polyglycérols récupéré est de 410 g et l'analyse du produit est la suivante : (% en poids)

| | | |
|---|---|---|
| Glycérine | % | 27,10 |
| Diglycérols | % | 32,15 |
| Triglycérols | % | 18,65 |
| Tétraglycérols | % | 9,30 |
| Pentaglycérols | % | 5,30 |
| Hexaglycérols | % | 2,00 |
| Cycliques | % | 5,50 |

### EXEMPLE 3

### Fabrication de polyglycérols

On répète l'exemple 1, mais on poursuit la réaction de polycondensation pendant 3 heures 15 à 265°C de manière qu'on recueille 79 cm3 d'eau. Puis on élimine l'eau résiduelle du mélange réactionnel en maintenant un vide dynamique, tout en refroidissant le mélange à environ 220°C en moins de 5 minutes ; puis on ramène la température à 150°C et on poursuit la procédure de l'exemple 1.

La composition du mélange de polyglycérols obtenu est donnée ci-après (% en poids).

| | | |
|---|---|---|
| Glycérine | % | 2,38 |
| Diglycérols | % | 19,96 |
| Triglycérols | % | 25,57 |
| Tétraglycérols | % | 11,59 |
| Pentaglycérols | % | 10,35 |
| Hexaglycérols | % | 6,94 |
| Heptaglycérols | % | 4,15 |
| Octaglycérols | % | 3,17 |
| Nonaglycérols | % | 1,57 |
| Décaglycérols | % | 0,77 |
| Cycliques variés | % | 13,55 |

Cette composition montre une très faible teneur résiduelle en glycérine.

### EXEMPLE 4 (Comparatif)

### Fabrication de polyglycérols.

Dans cet exemple, au lieu d'ajouter la soude totale après évaporation de l'eau, on ajoute directement la soude au départ.

A 800 g de glycérine sulfatée, titrant 65 % en poids de glycérol, on ajoute 5,4 g de soude en écailles. La solution noircit immédiatement même à 20 °C. On chauffe pour distiller l'eau. Un mousse importante se forme pendant la distillation de l'eau. On obtient un polyglycérol en cassant les mousses de temps en temps par un balayage d'argon jusqu'à 150 °C. Le produit obtenu correspond du point de vue analytique à celui fabriqué dans l'exemple 1. Le poids récupéré après passage sur résine est de 430,8 g de polyglycérol.

Dans cet exemple, il a fallu interrompre plusieurs fois le chauffage pour casser les mousses bien que l'on ait utilisé une colonne à reflux conséquente.

### EXEMPLE 5 (comparatif)

Au lieu d'ajouter de la soude dans la solution de glycérine sulfatée, on ajoute de la glycérine basique contenant des savons.

La quantité de savons correspond approximativement en ions sodium à 0,5 % en poids de soude. Malgré cet excès de soude présent sous forme de savons, on constate à 160 °C un commencement de décomposition de la glycérine en acroléïne. Cette décomposition est telle, qu'il a fallu arrêter le chauffage de la solution. On n'a donc pas pu fabriquer de polyglycérol.

Dans cet essai, la glycérine sulfatée mal neutralisée au départ a neutralisé la soude. Il restait dans la solution du sulfate acide de sodium.

### EXEMPLE 6

### Fabrication d'esters de polyglycérols.

Sur un polyglycérol fabriqué selon l'exemple 2, on ajoute avant de neutraliser les sels à 200 °C un acide gras de type acide 2-éthylhexanoïque de manière à avoir un excès d'acide par rapport aux hydroxyles mesurés par indice d'hydroxyle. La réaction se fait avec un "Dean and Stark" et dure 3 heures. Avec un indice d'hydroxyle de 1150 pour un polyglycérol, on utilise pour une substitution totale plus de 5 moles d'acide, la stoéchiométrie étant de 4 moles pour 200 g de polyglycérols. Ceci correspond à 5 x 144 = 720 g d'acide. On obtient 60 g d'eau et une partie de l'acide s'évapore. La substitution n'étant pas complète, on achève la réaction en acétylant le produit par de l'anhydride acitique,après avoir d'abord ajouté un peu d'acide acétique pour déplacer le savon formé.

A la fin de la réaction, on lave le produit à l'eau. Après 2 lavages, la plus grande partie des sels est éliminée.

Pour éliminer les traces, on passe sur une résine cationique puis sur une résine anionique en présence d'heptane.

### EXEMPLE 7

On chauffe 500 g d'une glycérine contenant en 8% poids de sel (NaCl) et 10 % d'eau, avec 1 % de soude concentrée introduite à froid. Après formation des polyglycérols à 270 °C, on obtient une solution de couleur légèrement jaune, que l'on peut traiter pour obtenir des polyglycérols, selon le procédé décrit dans l'exemple 2 puis pour obtenir des esters de polyglycérols selon le procédé décrit dans l'exemple 6.

## Revendications

1. Procédé pour l'obtention d'un mélange de polyglycérols dans lequel on effectue la condensation du glycérol contenu dans une matière première glycérineuse par chauffage en présence d'au moins un catalyseur basique, **caractérisé en ce que** ladite matière première glycérineuse comprend de la glycérine brute, contient au moins un sel minéral et ne présente pas un pH acide.

2. . Procédé selon la revendication 1, **caractérisé en ce que** ledit sel minéral est un sulfate, un chlorure ou un phosphate de métal alcalin.

3. Procédé selon la revendication 1 ou 2, **caractérisé en ce que** le dit catalyseur basique est choisi parmi la soude, la potasse, les carbonates et les alcoolates des métaux alcalins.

4. Procédé selon l'une quelconque des revendications 1 à 3, **caractérisé par le fait que**, pour réaliser la condensation du glycérol, on élimine l'eau de solution de ladite matière première glycérineuse en la chauffant jusqu'à une température d'environ 220 à 230°C, et qu'on en élimine l'eau de condensation en chauffant à une température de 230 à 270°C, puis on refroidit le mélange réactionnel à 150°C ou en dessous et on le neutralise.

5. Procédé selon la revendication 4, **caractérisé en ce que**, en fin de condensation, on élimine au maximum l'eau de condensation et on refroidit le mélange réactionnel en quelques minutes.

6. Procédé selon la revendication 5, **caractérisé en ce que** l'on élimine l'eau de condensation en appliquant un vide dynamique ou par entraînement au moyen d'un gaz inerte.

7. Procédé selon l'une quelconque des revendications 1 à 6, **caractérisé par le fait qu'**on élimine la plus grande partie de l'eau de solution par chauffage avant d'introduire le catalyseur basique.

8. Procédé selon l'une quelconque des revendications 1 à 7, **caractérisé par le fait que** l'on purifie le mélange de polyglycérols formé en neutralisant les ions alcalins provenant du catalyseur basique et des sels présents dans la matière première glycérineuse par l'addition d'acide sulfurique, qui produit un sulfate, on ajoute un alcool dans lequel ledit sulfate insoluble précipite, on filtre le sulfate précipité et l'on passe le mélange de polyglycérols ainsi purifié sur une résine cationique et sur une résine anionique en présence d'eau.

9. Procédé de préparation d'un ester de polyglycérols **caractérisé par le fait qu'**on prépare un mélange de polyglycérols en effectuant la condensation du glycérol contenu dans une matière première glycérineuse comprenant de la glycérine brute, contenant au moins un sel minéral et ne présentant pas un pH acide, par chauffage en présence d'au moins un catalyseur basique, on traite ledit mélange de polyglycérols avec un acide monocarboxylique aliphatique, linéaire ou ramifié, particulièrement à longue chaîne, un ester d'un tel acide et d'un monoalcool aliphatique ou une huile, à une température de 170 à 250°C et on élimine l'eau, le monoalcool ou le glycérol formé.

10. Procédé selon la revendication 9, **caractérisé par le fait qu'**on élimine les ions alcalins provenant des catalyseurs et/ou des sels initialement présents par addition d'un acide fort en milieu hydrocarboné ou alcoolique, on filtre les sels insolubles formés et on recueille la phase ester de polyglycérols obtenue.

11. Procédé selon la revendication 10, **caractérisé en ce que** l'on purifie ladite phase ester de polyglycérols en en séparant par décantation les polyglycérols qui n'ont pas réagi.

12. Procédé selon la revendication 9, **caractérisé par le fait que** l'on lave l'ester de polyglycérols à l'eau.

13. Procédé selon l'une quelconque des revendications 1 à 12, **caractérisé par le fait que**, après formation du mélange de polyglycérols et neutralisation de leur basicité, on distille ledit mélange sous pression réduite pour en éliminer la glycérine qui n'a pas réagi et certains composés cycliques.

## Claims

1. A process for producing a mixture of polyglycerols, in which the glycerol contained in a glycerin-containing starting material is condensed by heating in the presence of at least one basic catalyst, **characterized in that** said glycerin-containing starting material comprises unrefined glycerin, contains at least one mineral salt and does not have an acidic pH.

2. A process according to claim 1, **characterized in that** said mineral salt is an alkali metal sulphate, a chloride or a phosphate.

3. A process according to claim 1 or claim 2, **characterized in that** said basic catalyst is selected from sodium hydroxide, potassium hydroxide and alkali metal carbonates and alcoholates.

4. A process according to any one of claims 1 to 3, **characterized in that** to carry out the glycerol condensation, water of solution is eliminated from said glycerin-containing starting material by heating it to a temperature of about 220°C to 230°C, and the water of condensation is eliminated by heating to a temperature of 230°C to 270°C, then the reaction mixture is cooled to 150°C or below and it is neutralized.

5. A process according to claim 4, **characterized in that** at the end of condensation, the maximum water of condensation is eliminated and the reaction mixture is cooled in a few minutes.

6. A process according to claim 5, **characterized in that** the water of condensation is eliminated by applying a dynamic vacuum or by entrainment in an inert gas.

7. A process according to any one of claims 1 to 6, **characterized in that** the majority of the water of solution is eliminated by heating before introducing.the basic catalyst.

8. A process according to any one of claims 1 to 7, **characterized in that** the mixture of polyglycerols formed is purified by neutralizing the alkali ions deriving from the basic catalyst and salts present in the glycerin-containing starting material by adding sulphuric acid, which produces a sulphate, an alcohol is added in which said insoluble sulphate precipitates, the precipitated sulphate is filtered and the mixture of purified polyglycerols is passed over a cationic resin and over an anionic resin in the presence of water.

9. A process for preparing a polyglycerol ester, **characterized in that** a mixture of polyglycerols is prepared by condensing glycerol contained in a glycerin-containing starting material comprising unrefined glycerin, containing at least one mineral salt and not having an acidic pH, by heating in the presence of at least one basic catalyst, said mixture of polyglycerols is treated with a linear or branched aliphatic, particularly long-chain, monocarboxylic acid, an ester of said acid and an aliphatic mono-alcohol or an oil, at a temperature of 170°C to 250°C, and the water, mono alcohol or glycerol formed are eliminated.

10. A process according to claim 9, **characterized in that** the alkali ions deriving from the catalysts and/or the salts initially present are eliminated by adding a strong acid in a hydrocarbon or alcoholic medium, the insoluble salts formed are filtered and the polyglycerol ester phase obtained is recovered.

11. A process according to claim 10, **characterized in that** said polyglycerol ester phase is purified by separating unreacted polyglycerols by decanting.

12. A process according to claim 9, **characterized in that** the polyglycerol ester is washed with water.

13. A process according to any one of claims 1 to 12, **characterized in that** after forming the mixture of polyglycerols and neutralizing their basicity, said mixture is distilled under reduced pressure to eliminate the unreacted glycerin and certain cyclic compounds.

## Patentansprüche

1. Verfahren zum Erhalten einer Polyglycerolmischung, bei dem die Kondensation des Glycerols durch Erwärmen in Gegenwart von mindestens einem basischen Katalysator ausgeführt wird, **dadurch gekennzeichnet, dass** der glycerinhaltige Rohstoff, der rohes Glycerin umfasst, mindestens ein Mineralsalz enthält, und keinen sauren pH-Wert aufweist.

2. Verfahren nach Anspruch 1, **dadurch gekennzeichnet, dass** das Mineralsalz ein Alkalimetallsulfat, ein Alkalimetallchlorid oder ein Alkalimetallphosphat ist.

3. Verfahren nach Anspruch 1 oder 2, **dadurch gekennzeichnet, dass** der basische Katalysator aus Natriumcarbonat, Kaliucarbonat, Alkalimetallcarbonaten und Alkalimetallalkoholaten ausgewählt ist.

4. Verfahren nach einem der Ansprüche 1 bis 3, **gekennzeichnet durch** die Tatsache, dass, um die Kondensation des Glycerols zu realisieren, das Lösungswasser des glycerinhaltigen Rohstoffs entfernt wird, indem es bis auf eine Temperatur von etwa 220 bis 230 °C erwärmt wird, und das Kondensationswasser entfernt wird, indem es auf eine Temperatur von 230 bis 270 °C erwärmt wird, dann wird die Reaktionsmischung auf 150 °C oder darunter abgekühlt und sie wird neutralisiert.

5. Verfahren nach Anspruch 4, **dadurch gekennzeichnet, dass** am Ende der Kondensation das Kondensationswasser weitmöglichst entfernt wird, und dass die Reaktionsmischung innerhalb einiger Minuten abgekühlt wird.

6. Verfahren nach Anspruch 5, **dadurch gekennzeichnet, dass** das Kondensationswasser entfernt wird, indem ein dynamisches Vakuum angewendet wird, oder durch Mitreißen mittels eines inerten Gases.

7. Verfahren nach irgendeinem der Ansprüche 1 bis 6, **gekennzeichnet durch** die Tatsache, dass der größte Teil des Lösungswassers **durch** Erwärmen entfernt wird, bevor der basische Katalysator eingeführt wird.

8. Verfahren nach irgendeinem der Ansprüche 1 bis 7, **gekennzeichnet durch** die Tatsache, dass die gebildete Polyglycerolmischung gereinigt wird, indem die alkalischen Ionen, die von dem basischen Katalysator und den Salzen stammen, die in dem glycerinhaltigen Rohstoff vorhanden sind, **durch** Zugabe von Schwefelsäure neutralisiert werden, was ein Sulfat produziert, es wird ein Alkohol zugegeben, in dem das unlösliche Sulfat präzipitiert, das präzipitierte Sulfat wird gefiltert und die so gereinigte Polyglycerolmischung in Gegenwart von Wasser auf ein kationisches Harz und auf ein anionisches Harz gegeben.

9. Verfahren zur Herstellung eines Polyglycerolesters, **gekennzeichnet durch** die Tatsache, dass eine Polyglycerolmischung hergestellt wird, indem die Kondensation des Glycerols ausgeführt wird, das in einem glycerinhaltigen Rohstoff enthalten ist, der rohes Glycerin umfasst, der mindestens ein Mineralsalz enthält und keinen sauren pH-Wert aufweist, **durch** Erwärmen in Gegenwart von mindestens einem basischen Katalysator, die Polyglycerolmischung mit einer linearen oder verzweigen aliphatischen, insbesondere langkettigen Monocarbonsäure, einem Ester einer solchen Säure und einem aliphatischen Monoalkohol, oder einem Öl, bei einer Temperatur von 170 bis 250 °C behandelt wird, und das Wasser, der Monoalkohol oder das gebildete Glycerol entfernt wird.

10. Verfahren nach Anspruch 9, **gekennzeichnet durch** die Tatsache, dass die alkalischen Ionen, die aus den Katalysatoren und/oder Salzen stammen, die anfangs vorhanden sind, **durch** Zugabe einer starken Säure in Kohlenwasserstoff- oder Alkoholmedium entfernt werden, dass die gebildeten unlöslichen Salze gefiltert werden, und dass die Esterphase des erhaltenen Polyglycerols gesammelt wird.

11. Verfahren nach Anspruch 10, **dadurch gekennzeichnet, dass** die Polyglycerolesterphase gereinigt wird, indem die Polyglycerole, die nicht umgesetzt wurden, durch Dekantierung abgeschieden werden.

12. Verfahren nach Anspruch 9, **gekennzeichnet durch** die Tatsache, dass der Polyglycerolester mit Wasser gewaschen wird.

13. Verfahren nach irgendeinem der Ansprüche 1 bis 12, **gekennzeichnet durch** die Tatsache, dass, nach der Bildung der Polyglycerolmischung und der Neutralisierung ihrer Basizität, die Mischung unter verringertem Druck destilliert wird, um das Glycerin, das nicht umgesetzt wurde, und bestimmte cyclische Verbindungen daraus zu entfernen.
